# EUROPEAN PATENT APPLICATION

(11) **EP 3 936 581 A1**
(43) Date of publication of application: **12.01.2022**
(21) Application number: 20766738.7
(22) Date of filing: 02.03.2020
(51) Int. Cl.: C09D 129/04, A61K 9/32, A61K 45/00, A61K 47/32, A61K 47/34, A61K 47/36, C09D 7/43, C09D 7/63, C09D 7/65, C09D 171/00

(54) **FILM COATING COMPOSITION AND SOLID PREPARATION**

(30) Priority: 04.03.2019 JP 2019038589
(71) Applicant: Sawai Pharmaceutical Co., Ltd., Yodogawa-ku Osaka-shi Osaka 532-0003 (JP)
(72) Inventor: IZUI Wataru, Osaka City, Osaka 532-0003 (JP); NAKAMICHI Katsuki, Osaka City, Osaka 532-0003 (JP); MATSUKAWA Takuya, Osaka City, Osaka 532-0003 (JP); KIKUOKA Hiroaki, Osaka City, Osaka 532-0003 (JP)
(74) Representative: dompatent von Kreisler Selting Werner - Partnerschaft von Patent- und Rechtsanwälten mbB
(86) International application number: PCT/JP2020/008709
(87) International publication number: WO 2020/179736

(57) **Abstract**

The present invention provides a film coating composition for a solid preparation that is easy to take. The present invention also provides a solid preparation that is easy to take. A film coating composition according to an embodiment of the present invention contains a polyvinyl alcohol-polyethylene glycol graft copolymer and a thickening agent. The thickening agent may be composed of one or more substances that are selected from the group consisting of xanthan gum, locust bean gum, pectin, carrageenan, guar gum, gellan gum, and carboxy vinyl polymer.

## Description

### TECHNICAL FIELD

The present invention relates to a film coating composition and a solid preparation.

### BACKGROUND OF THE INVENTION

Many solid preparations to be administered orally are prepared by coating an outer surface of the preparation with a film to prevent unnecessary exposure of an active ingredient and mask the bitterness and the like of the active ingredient. In addition, from the viewpoint of ingesting ability, for example, a preparation in which a plain tablet is coated with a gelling agent has been studied in recent years.

For example, as a technique which improves the cohesiveness of tablets in the oral cavity and makes it easier to swallow, Patent Literature 1 describes a coating composition including a) a first thickener selected from a group consisting of carboxy vinyl polymer and sodium alginate; b) polyvalent metal compound; c) at least one or more kinds of second thickeners selected from a group consisting of xanthan gum, guar gum, and sodium alginate, which are 10 to 40% by mass in all components excluding a solvent (when the first thickener is sodium alginate, the second thickener is not sodium alginate); d) hydroxypropyl methylcellulose, which is 5 to 35% by mass in all components excluding the solvent; and e) sugar or sugar alcohol with a solubility of 30g or more at 20°C in 100g of water, which is 10 to 50% by mass in all components excluding the solvent; and f) alcohol, or water and alcohol as the solvent.

Patent Literature 2 describes, to impart excellent moldability (hardness), slipperiness, and the like, a particle composition for easy-to-take solid preparation containing a sugar alcohol, a gelling agent that exhibits slipperiness upon contact with water, and the particle composition being characterized in part or all of the surface of the particle composition is covered by the gelling agent. Patent Literature 3 describes a film coating composition in powder form, comprising a water-soluble polymer and guar gum to improve swallowability, wherein the guar gum has a minimum viscosity of about 700 cP when dissolved in water at a 1%w/w concentration for 2 hours as measured on a Brookfield RVT at 25°C, and the guar gum gives a weight gain of at least about 0.25% by weight to a plain tablet coated with an aqueous dispersion which contains the film coating composition, and the film coating composition includes at least one of a) a static friction coefficient of less than about 3; or b) a kinetic friction coefficient of less than 5.

### CITATION LIST

### PATENT LITERATURE

Patent Literature 1: Japanese Patent No. 5426018
Patent Literature 2: International publication No. WO2017/057147
Patent Literature 3: International publication No. WO2018/026596

### BRIEF DESCRIPTION OF DRAWINGS

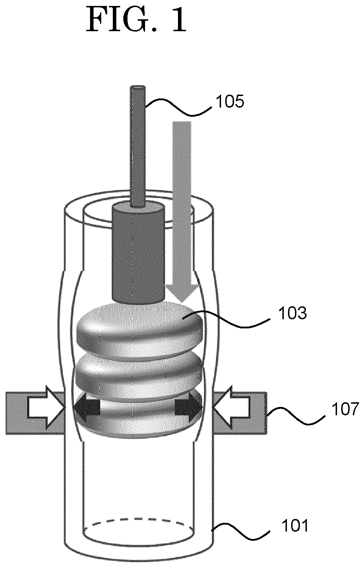

FIG. 1 is a schematic diagram showing a measurement method of the maximum stress according to an example of the present invention.

### SUMMARY OF THE INVENTION

### TECHNICAL PROBLEM

An embodiment of the present invention provides a film coating composition for an easy-to-take solid preparation. Also, an embodiment of the present invention provides an easy-to-take solid preparation.

### SOLUTION TO PROBLEM

According to an embodiment of the present invention, there is provided a film coating composition including polyvinyl alcohol-polyethylene glycol graft copolymer, and a thickening agent.

The thickening agent may be one or more substances selected from a group consisting of xanthan gum, locust bean gum, pectin, carrageenan, guar gum, gellan gum, and carboxy vinyl polymer.

According to an embodiment of the present invention, there is provided a solid preparation including a plain tablet containing an active ingredient, and a coating layer arranged outside the plain tablet, and the coating layer composed of the film coating composition according to claim 1.

### EFFECT OF INVENTION

According to an embodiment of the present invention, a film coating composition for an easy-to-take solid preparation can be provided. In addition, according to an embodiment of the present invention, an easy-to-take solid preparation can be provided.

### DESCRIPTION OF EMBODIMENTS

### [Film coating composition]

A film coating composition according to an embodiment of the present invention includes polyvinyl alcohol-polyethylene glycol graft copolymer and a thickening agent. The thickening agent may exemplify, but is not limited to, xanthan gum, locust bean gum, pectin, carrageenan, guar gum, gellan gum, and carboxy vinyl polymer, and the like. These thickening agents may be used in combination of one or more of them as appropriate. In an embodiment, the thickening agent may be one or more substances selected from a group consisting of xanthan gum, carrageenan, and carboxy vinyl polymer.

### [Solid preparation]

In an embodiment, a film coating composition may be used to coat an outer surface of a plain tablet containing an active ingredient. That is, a coating layer in which the film coating composition is solidified is arranged on the outside of the plain tablet. The plain tablet to be coated is not particularly limited, and is not limited to a circular tablet, and may be an irregular-shaped tablet such as an ellipse or a polygon. Also, a tablet having a general size may be used, and a mini-tablet may be used. Here, the tablet having a general size may have, for example, a diameter or a major diameter of 5 mm or more and 20 mm or less. The mini-tablet has a diameter or a major diameter of less than 5 mm and can be, for example, 1 mm or more and 4 mm or less.

In general, it is known that the smaller the size of a tablet, the easier it is to swallow, and it is said that if it exceeds 8 mm in diameter, it is difficult to swallow. In view of ease of handling, it is also known that a tablet size suitable for elderly people with impaired swallowing function is 7 to 8 mm in diameter in the case of a circular tablet. Even if the solid preparation coated with the film coating composition of the present invention has a size larger than this, when it is wetted with water such as saliva, the surface becomes easily gelled and slippery, so that resistance when passing through the throat is reduced and easily swallowed, and in addition, it is also possible to mask the taste such as drug-derived bitterness. In addition, the film coating composition of the present invention can be applied not only to a tablet having a commonly used size but also to a mini-tablet having a diameter of about 1 to 4 mm. The mini-tablet has recently attracted attention as a dosage form that is easy to adjust in dosage and is easy to swallow even for children and the elderly. A mini-tablet coated with the film coating composition of the present invention does not stick in the oral cavity, and a plurality of tablets can be taken together. In addition, in an embodiment, the film coating composition of the present invention is easy to adjust the pharmaceutical properties such as a disintegration of a solid preparation, a dissolution rate of a film coating layer, or a degree of masking a taste while having a simple preparation configuration, and it is possible to conveniently produce a desired solid preparation.

### [Method for producing film coating composition]

The film coating composition according to an embodiment of the present invention can be produced by a known producing method. In an embodiment, the film coating composition may be prepared, for example, by dissolving or dispersing polyvinyl alcohol-polyethylene glycol graft copolymer, a thickening agent, and optionally a pharmaceutically acceptable additive agent in a solvent. The solvent to be used may be any solvent commonly used for film coating, and includes, for example, alcohol such as ethanol and isopropanol, water, or a mixture thereof. Amount of the thickening agent to be used can be appropriately increased or decreased depending on a type of thickening agent. However, when the amount of the thickening agent is large, the viscosity of the film coating composition becomes too high, and it may be physically difficult to apply a coating to the plain tablet. Accordingly, although suitable amount of the thickening agent varies depending on the type thereof, it is preferably smaller than that of the polyvinyl alcohol-polyethylene glycol graft copolymer, and more preferably 1% by weight or more and 20% by weight or less, 1% by weight or more and 10% by weight or less, or alternatively 2% by weight or more and 7% by weight or less with respect to the polyvinyl alcohol-polyethylene glycol graft copolymer.

### [Method for producing solid preparation]

Using the film coating composition according to an embodiment of the present invention, a solid preparation having a coating layer can be produced by a known producing method. For example, a coating device may be used to spray the film coating composition onto the plain tablet to apply a coating, and the solvent may be removed to produce a solid preparation having a coating layer.

### EXAMPLES

### [Comparative Example 1 (plain tablet)]

158.0 kg of lactose hydrate, 39.6 kg of crystalline cellulose, and 2.4 kg of magnesium stearate were mixed in a mixer, and the obtained mixed powder was tableted by a rotary tableting machine to obtain tablets (265 mg per 1 tablet, ϕ (diameter) 9 mm, the thickness of 4.5 mm).

### [Examples 1 to 15]

As Examples 1 to 15 of the present invention, polyvinyl alcohol-polyethylene glycol graft copolymer (Kollicoat (registered trademark) IR, BASF), a thickening agent, and other additive agents were dissolved and dispersed in a mixture of water and ethanol (8:2) to prepare a film coating composition. As an example, a thickening agent selected from xanthan gum, i carrageenan, K carrageenan, locust bean gum, LM pectin, HM pectin, guar gum, gellan gum, and carboxy vinyl polymer was examined. The compositions of the film coating compositions of Examples 1 to 15 are shown in Tables 1 to 3. Using a coating device (LABO, Freund Corporation), a film coating composition was sprayed onto the plain tablet (ϕ 9 mm) obtained in Comparative Example 1 to be equivalent to 3 w/w% as a solid content per tablet to obtain solid preparations of Examples 1 to 15.

The compositions of the film coating compositions of Examples 1 to 15 are shown in Tables 1 to 3.

**[Table 1]**

| | Example 1 | Example 2 | Example 3 | Example 4 | Example 5 |
|---|---|---|---|---|---|
| Polyvinyl alcohol-polyethylene glycol graft copolymer | 9.5 | 9.75 | 9.5 | 7.75 | 7.5 |
| Xanthan gum | 0.5 | 0.25 | 0.25 | 0.25 | 0.5 |
| Locust bean gum | - | - | 0.25 | - | - |
| Titanium oxide | - | - | - | 1 | 1 |
| Talc | - | - | - | 1 | 1 |
| (water/ethanol=8/2) | (100) | (100) | (100) | (100) | (100) |
| Total | 10 | 10 | 10 | 10 | 10 |
| Maximum stress (slipperiness, N) | 2.8 | 3.1 | 2.5 | 2.9 | 2.6 |
| Disintegration Time (sec) | 354 | 313 | 298 | 330 | 407 |
| Dissolution Time of film coating layer (sec) | 239 | 198 | 183 | 215 | 292 |

| | | | | | |
|---|---|---|---|---|---|
| *The unit in the table is w/w%. | | | | | |

**[Table 2]**

| | Example 6 | Example 7 | Example 8 | Example 9 | Example 10 |
|---|---|---|---|---|---|
| Polyvinyl alcohol-polyethylene glycol graft copolymer | 9.5 | 9.5 | 9.5 | 7.5 | 9.75 |
| I carrageenan | 0.5 | - | - | - | - |
| K carrageenan | - | 0.5 | - | - | - |
| locust bean gum | - | - | - | - | 0.25 |
| carboxy vinyl polymer | - | - | 0.5 | 0.5 | - |
| Titanium oxide | - | - | - | 1 | - |
| Talc | - | - | - | 1 | - |
| (water/ethanol=8/2) | (100) | (100) | (100) | (100) | (100) |
| Total | 10 | 10 | 10 | 10 | 10 |
| Maximum stress (slipperiness, N) | 2.6 | 2.7 | 2.5 | 3.1 | 2.5 |
| Disintegration Time (sec) | 258 | 272 | 817 | 626 | 140 |
| Dissolution Time of film coating layer (sec) | 143 | 157 | 702 | 511 | 25 |

| | | | | | |
|---|---|---|---|---|---|
| *The unit in the table is w/w%. | | | | | |

**[Table 3]**

| | Example 11 | Example 12 | Example 13 | Example 14 | Example 15 |
|---|---|---|---|---|---|
| Polyvinyl alcohol-polyethylene glycol graft copolymer | 9.5 | 9.5 | 9.5 | 9.5 | 9.5 |
| locust bean gum | 0.5 | - | - | - | - |
| LM pectin | - | 0.5 | - | - | - |
| HM pectin | - | - | 0.5 | - | - |
| guar gum | - | - | - | 0.5 | - |
| gellan gum | - | - | - | - | 0.5 |
| (water/ethanol=8/2) | (100) | (100) | (100) | (100) | (100) |
| Total | 10 | 10 | 10 | 10 | 10 |
| Maximum stress (slipperiness, N) | 2.5 | 2.6 | 3.4 | 2.8 | 2.7 |
| Disintegration Time (sec) | 147 | 156 | 181 | 140 | 137 |
| Dissolution Time of film coating layer (sec) | 32 | 41 | 66 | 25 | 22 |

| | | | | | |
|---|---|---|---|---|---|
| *The unit in the table is w/w%. | | | | | |

### [Comparative Examples 2 to 7]

As Comparative Example 2, a film coating composition was produced using only polyvinyl alcohol-polyethylene glycol graft copolymer without a thickening agent. As Comparative Example 3, a film coating composition was produced using polyvinyl alcohol-polyethylene glycol graft copolymer and a thickening agent in the same amount. As Comparative Examples 4 to 7, film coating compositions were produced using hypromellose (hereinafter also referred to as HPMC) or hydroxypropyl cellulose (hereinafter also referred to as HPC) in place of the polyvinyl alcohol-polyethylene glycol graft copolymer used in Examples 1 to 15. The film coating compositions of Comparative Examples 4 to 7 were produced using the same producing method as in Examples 1 to 15, except that the polyvinyl alcohol-polyethylene glycol graft copolymer used in Examples 1 to 15 was substituted to HPMC (TC-5 (registered trademark) M, Shin-Etsu Chemical Co., Ltd.) or HPC (SSL, Shin-Etsu Chemical Co., Ltd.), and other additive agents were added. The compositions of the film coating compositions of Comparative Examples 2 to 7 are shown in Tables 4 to 5. The film coating compositions of Comparative Examples 3 to 5 had extremely high viscosity and difficult to prepare, and it was impossible to produce a solid preparation. Using the coating device (LABO, Freund Corporation), a film coating composition was sprayed onto the plain tablet (ϕ 9 mm) obtained in Comparative Example 1 to be equivalent to 3 w/w% as a solid content per tablet to obtain solid preparations of Comparative Examples 2, 6, and 7.

### [Comparative Example 8]

As Comparative Example 8, a film coating composition was produced in accordance with Example 1 described in Patent Literature 1. Specifically, HPMC, HPC, carboxy vinyl polymer, calcium chloride dihydrate, jet milled xanthan gum and jet milled erythritol were dispersed in ethanol to prepare a film coating composition of Comparative Example 8. The composition of the film coating composition of Comparative Example 8 is shown in Table 6. Using the coating device (LABO, Freund Corporation), the film coating composition was sprayed onto the plain tablet (ϕ 9 mm) to be equivalent to 3 w/w% as a solid content per tablet to obtain a solid preparation of Comparative Example 8. In the film coating composition of Comparative Example 8, it took more time and labor to perform jet milling when producing, and the operation was complicated. There were safety concerns such as explosion hazard because it was necessary to use 100% ethanol, which is a highly volatile organic solvent.

The compositions of the film coating compositions of Comparative Examples 2 to 8 are shown in Tables 4 to 6.

**[Table 4]**

| | Comparative Example 1 | Comparative Example 2 | Comparative Example 3 | Comparative Example 4 |
|---|---|---|---|---|
| Polyvinyl alcohol-polyethylene glycol graft copolymer | - | 10 | 5 | - |
| hypromellose | - | - | - | 9.5 |
| Xanthan gum | - | - | 5 | 0.5 |
| (water/ethanol=8/2) | | (100) | (100) | (100) |
| Total | 0 | 10 | 10 | 10 |
| Maximum stress (slipperiness, N) | 5.0 | 2.3 | - | - |
| Disintegration Time | 115 | 126 | - | - |
| (sec) | | | | |
| Dissolution Time of film coating layer (sec) | - | 11 | - | - |

| | | | | |
|---|---|---|---|---|
| *The unit in the table is w/w%. | | | | |

**[Table 5]**

| | Comparative Example 5 | Com parative Example 6 | Com parative Example 7 |
|---|---|---|---|
| hypromellose | - | 10 | 10 |
| hydroxypropyl cellulose | 9.5 | - | - |
| Xanthan gum | 0.5 | - | - |
| Macrogol 6000 | - | 1 | 1 |
| Titanium oxide | - | - | 1 |
| Total | - | - | 1 |
| (water/ethanol=8/2) | (100) | (100) | (100) |
| Sum | 10 | 11 | 13 |
| Maximum stress (slipperiness, N) | - | 4.5 | 4.3 |
| Disintegration Time (sec) | - | 323 | 302 |
| Dissolution Time of film coating layer (sec) | - | 208 | 187 |

| | | | |
|---|---|---|---|
| *The unit in the table is w/w%. | | | |

**[Table 6]**

| | Com parative Example 8 |
|---|---|
| hypromellose | 1.8 |
| hydroxypropyl cellulose | 1.2 |
| carboxy vinyl polymer | 1.5 |
| Xanthan gum (jet milled) | 3 |
| calcium chloride dihydrate | 0.15 |
| Erythritol (jet milled) | 4.2 |
| (ethanol) | (80) |
| Total | 11.85 |
| Maximum stress (slipperiness, N) | 2.3 |
| Disintegration Time (sec) | Over 1800 |
| Dissolution Time of film coating layer (sec) | Over 1800 |

| | |
|---|---|
| *The unit in the table is w/w%. | |

### [Sensory Test]

Three subjects (adult males) took 1 tablet each of plain tablets or solid preparations of the above-mentioned Examples and Comparative Examples, and the feeling of taking them was confirmed. The plain tablet of Comparative Example 1 had strong resistance to the throat and was difficult to swallow when taken. The solid preparation of Comparative Example 2 coated with the film coating composition using only polyvinyl alcohol-polyethylene glycol graft copolymer without thickening agent, immediately dissolved the film coating layer after being put into the oral cavity, and the plain tablet surface was exposed. It was difficult to swallow, similar to Comparative Example 1, and it was presumed that it was also difficult to apply the solid preparations to a tablet which required masking of taste. The solid preparations of Comparative Examples 6 and 7 coated with a general film coating composition using a commonly used water-soluble polymer without thickening agent had a feeling of sticking in the oral cavity when taken.

### [Measurement of maximum stress]

For the plain tablets or solid preparations of the examples and comparative examples described above, the slipperiness was evaluated by measuring the maximum stress when passing through a silicon tube. FIG. 1 is a schematic diagram showing a measurement method of the maximum stress according to an example of the present invention. A small tabletop tester (SHIMADZU EZ-LX) manufactured by Shimadzu Corporation was used to measure the maximum stress. A silicone tube 101 (ϕ 9 mm) was filled with 3 tablets of ϕ 9 mm plain tablet or solid preparation 103 soaked in water for 2 seconds and extruded from above the silicon tube 101 using a cylindrical plunger 105 with a diameter of 5 mm. The lower portion of the silicon tube 101 is fixed with a stainless steel plate 107 with a hole of the same size as the outer diameter of the silicon tube 101, and the maximum stress when passing through the fixed portion was measured. The maximum stress was determined as the average value of the three measurements. The maximum stresses of the measured plain tablets or solid preparations in the Examples and Comparative Examples are shown in Tables 1 to 6, respectively. In the above measurement method using the ϕ 9 mm tablet, the solid preparations of Examples 1 to 15 using the film coating composition in the present invention resulted in the maximum stress of approximately 4N or less. This was a result that the slipperiness was better than that of the plain tablet of Comparative Example 1 that had strong resistance to the throat and was difficult to swallow and that of the solid preparation of Comparative Examples 6 and 7 with a general film coating that gave a feeling of sticking in the oral cavity in the sensory test.

### [Measurement of disintegration time and dissolution time of film coating layer]

Disintegration times were measured using a disintegration tester (NT-600, TOYAMA SANGYO CO., LTD.) in accordance with the Japanese Pharmacopoeia, 17th edition, General Tests, Disintegration Test. 900 mL of water maintained at 37°C ± 2.0°C was used as the test solution, and the time to the disintegration of 3 tablets was measured. Further, the difference between the measured disintegration time and the disintegration time in Comparative Example 1 (the plain tablet) was calculated as the dissolution time of the film coating layer. The disintegration times of the measured plain tablets or solid preparations in the Examples and Comparative Examples and the dissolution times of the film coating layers are shown in Tables 1 to 6, respectively. The disintegration time is preferably within 60 minutes, more preferably within 30 minutes, because it is not preferable to extremely extend the disintegration time by applying a film coating. The dissolution time of the film coating layer is preferably 20 seconds or more in view of the time required for the people to swallow, and 30 seconds or more in view of the people with impaired swallowing function. As a result, the dissolution time of the film coating layer in Comparative Example 2 was only 11 seconds, which was a result consistent with the result of the sensory test. Unlike the mini-tablet described in Patent Literature 1, it has been found in Comparative Example 8 that when applied to a tablet having a general size (ϕ 9 mm), the disintegration time becomes extremely slow.

### [Comparative Example 9 (tablet of mini-tablet)]

To a granulated product obtained by granulating and drying 500 parts by weight of levetiracetam as a bitter drug, 5 parts by weight of light anhydrous silicic acid, and 5 parts by weight of hydroxypropyl cellulose with 162 parts by weight of water using a fluidized bed granulator (MP-01, Powrex Corporation), 10.4 parts by weight of croscarmellose sodium, 11.2 parts by weight of hydroxypropyl cellulose, 5.4 parts by weight of light anhydrous silicic acid, and 0.6 parts by weight of magnesium stearate were added and mixed them, and the obtained mixed powder was tableted by a rotary tableting machine to obtain tablets (6.4 mg per 1 tablet, ϕ (diameter) 2 mm, thickness 2 mm).

### [Example 16]

Polyvinyl alcohol-polyethylene glycol graft copolymer (Kollicoat (registered trademark) IR, BASF), and xanthan gum as the thickening agent, titanium oxide, and talc were dissolved and dispersed in a mixture of water and ethanol (8:2) to prepare a film coating composition. Using the coating device (LABO, Freund Corporation), a film coating composition was sprayed onto the plain tablet of a mini-tablet obtained in Comparative Example 9 to be equivalent to 13 w/w% as a solid content per tablet to obtain a solid preparation of Example 16.

### [Comparative Example 10]

As Comparative Example 10, a film coating composition was prepared without using xanthan gum used in Example 16. Using the coating device (LABO, Freund Corporation), a film coating composition was sprayed onto the plain tablet of a mini-tablet obtained in Comparative Example 9 to be equivalent to 13 w/w% as a solid content per tablet to obtain a solid preparation of Comparative Example 10.

The compositions of the film coating compositions of Example 16 and Comparative Example 10 are shown in Table 7.

**[Table 7]**

| | Example 16 | Com parative Example 9 | Com parative Example 10 |
|---|---|---|---|
| Polyvinyl alcohol-polyethylene glycol graft copolymer | 9.5 | - | 10 |
| Xanthan gum | 0.5 | - | - |
| Titanium oxide | 1 | - | 1 |
| Talc | 1 | - | 1 |
| (water/ethanol=8/2) | (100) | - | (100) |
| Total | 12 | - | 12 |
| Maximum stress (slipperiness, N) | 4.3 | 14.1 | - |

| | | | |
|---|---|---|---|
| *The unit in the table is w/w%. | | | |

### [Sensory Test]

Three subjects (adult males) took 84 tablets each of plain tablets or solid preparations of Example 16 and Comparative Examples 9 to 10 described above, and the feeling of taking them was confirmed. In Example 16, it took 20 to 30 seconds to feel the drug-derived bitterness when taken, whereas in Comparative Examples 9 to 10, the bitter taste was experienced immediately after taking them.

In Example 16, the tablets did not stick in the oral cavity when taken, stick to each other, and were easy to take, but in Comparative Examples 9 to 10, the tablets did not stick to each other and were difficult to take.

### [Measurement of maximum stress]

For the plain tablet or solid preparation of Example 16 and Comparative Example 9 described above, slipperiness was evaluated by measuring the maximum stress when passing through the silicon tube. The small tabletop tester (SHIMADZU EZ-LX) manufactured by Shimadzu Corporation was used to measure the maximum stress. The silicon tube (ϕ 9 mm) was filled with 84 tablets of ϕ 2 mm plain tablet or solid preparation, 5 mL of water was dropped onto the tablets, and extruded from above the silicon tube by the method described above using the cylindrical plunger with a diameter of 5 mm. The maximum stress was determined as the average value of the three measurements. The maximum stresses of the measured plain tablets or solid preparations of Example 16 and Comparative Example 9 are shown in Table 7. The reason why the maximum stress is larger in the case of using a ϕ 2 mm solid preparation than in the case of using a ϕ 9 mm solid preparation is considered to be that the frictional force with respect to the silicon tube is larger due to the larger surface area. In the above measurement method using a ϕ 2 mm solid preparation, the solid preparation of Example 16 using the film coating composition in the present invention had a result that slipperiness was better than that of the plain tablet of Comparative Example 9 that did not stick to each other and were difficult to take, in the sensory test.

### [Description of Symbols]

- 101:: silicone tube, 103: plain tablet or solid preparation, 105: cylindrical plunger,
- 107:: plate

## Claims

1. A film coating composition comprising: polyvinyl alcohol-polyethylene glycol graft copolymer; and thickening agent.

2. A film coating composition according to claim 1, wherein the thickening agent is one or more substances selected from a group consisting of xanthan gum, locust bean gum, pectin, carrageenan, guar gum, gellan gum, and carboxy vinyl polymer.

3. A solid preparation comprising:
a plain tablet containing an active ingredient; and
a coating layer arranged outside the plain tablet, and the coating layer composed of the film coating composition according to claim 1.
